(19) Europäisches Patentamt
European Patent Office
Office européen des brevets

(11) Numéro de publication: **0 097 666 B1**

# (12) FASCICULE DE BREVET EUROPEEN

(45) Date de publication de fascicule du brevet: **19.08.92**

(51) Int. Cl.[5]: **C07H 19/20**, G01N 33/50, C12Q 1/00

(21) Numéro de dépôt: **83900049.4**

(22) Date de dépôt: **29.12.82**

(86) Numéro de dépôt internationale : **PCT/FR82/00222**

(87) Numéro de publication internationale : **WO 83/02276 (07.07.83 83/16)**

## (54) PROCEDE DE DOSAGE DE SUBSTANCES BIOLOGIOUES SUSCEPTIBLES DE FIXER DES PRODUITS DE DEGRADATION DE L'ACIDE ADENOSINE 5'-TRIPHOSPHORIOUE.

(30) Priorité: **29.12.81 FR 8124442**

(43) Date de publication de la demande: **11.01.84 Bulletin 84/02**

(45) Mention de la délivrance du brevet: **19.08.92 Bulletin 92/34**

(84) Etats contractants désignés: **BE CH DE GB LI**

(56) Documents cités:
**DE-A- 2 618 419**
**DE-A- 2 618 511**
**US-A- 4 255 566**

(73) Titulaire: **INSTITUT PASTEUR**
**28, rue du Docteur Roux**
**F-75715 Paris Cedex 15(FR)**

(72) Inventeur: **KOURILSKY, Philippe**
**207, rue de Vaugirard**
**F-75015 Paris(FR)**
Inventeur: **VINCENT, Christian**
**24, rue du Hameau**
**F-75015 Paris(FR)**
Inventeur: **TCHEN, Paul**
**18, rue du Télégraphe**
**F-92000 Nanterre(FR)**

(74) Mandataire: **Gutmann, Ernest et al**
**S.C. Ernest Gutmann - Yves Plasseraud 67, boulevard Haussmann**
**F-75008 Paris(FR)**

EP 0 097 666 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention est relative à un à un procédé de dosage de substances biologiques susceptibles, en présence de l'enzyme appropriée, d'incorporer un produit de dégradation de l'ATP, en d'autres termes l'acide adénosine 5'-monophosphate (AMP) ou de l'acide adénosine 5'-diphosphate (ADP).

Il est connu qu'une catégorie de substances biologiques, notamment certains antibiotiques, plus particulièrement ceux de la classe des aminoglycosides, sont capables, en présence d'enzymes spécifiques, d'incorporer de l'ADP ou de l'AMP, ces dernières molécules étant susceptibles d'être fournies à cet antibiotique à partir de l'ATP. Cette incorporation est schématisée par l'équation de réaction suivante, dans laquelle R-OH représente l'antibiotique :

$$\text{R-OH} + \text{ATP} \xrightarrow{\text{enzyme spécifique}} \text{R-O-AMP} + \text{PPi}$$

Les abréviations PPi (pyrophosphate) et Pi (phosphate) sont quelquefois utilisées en biologie pour désigner les dérivés "inorganiques" du phosphore qui sont libérés à l'occasion de réactions biologiques du type sus-indiqué.

Ce type de réaction est certes déjà utilisé dans des dosages de substances biologiques du type sus-indiqué, mais les moyens de révélation connus deviennent inopérants, lorsque ces substances biologiques ne sont présentes qu'en quantités infimes dans les échantillons étudiés.

L'invention a pour but de fournir un procédé suffisamment sensible mettant précisément en oeuvre la susdite réaction pour doser de telles substances biologiques, même lorsqu'elles n'existent dans les échantillons biologiques étudiés qu'en quantités infimes, notamment du nanogramme ou même beaucoup moins.

Le procédé selon l'invention met en oeuvre un réactif consistant dans un produit de modification de l'ATP, caractérisé par la fixation de façon covalente sur cet ATP d'une molécule chimique portant au moins un groupe non engagé dans la liaison covalente susdite et couplable directement ou indirectement avec une molécule ou un produit ayant une affinité spécifique pour ce groupe et permettant par conséquent la reconnaissance sélective des substances biolologiques susdites.

Des produits correspondant à ces réactifs ont déjà été décrits dans des demandes de brevet allemand n° 2.618.419 et n° 2.618.511, toutes deux déposées le 27 avril 1976 par la société MILES LABORATORIES, INC. Ils sont présentés comme constituant des conjugués formés entre une molécule déterminée, alors dénommée "ligand" et de l'ATP.

Selon ces demandes de brevets, ces composés peuvent être mis en oeuvre dans des procédés de dosage de ces ligands ou de dérivés de ces derniers, dans des solutions susceptibles de les contenir. Les procédés du genre en question comprennent alors la mise en réaction de l'ATP entrant dans la constitution desdits conjugués avec un substrat approprié (par exemple la luciférine réduite), le cas échéant en présence d'une enzyme jouant le rôle de catalyseur (par exemple la luciférase), à mesurer la variation de l'activité de l'ATP à l'égard du substrat, et à corréler la variation mesurée à l'existence et à la concentration du "ligand" dans le milieu étudié.

Le brevet américain n° 4.255.566 décrit également des conjugués du type sus-indiqué. L'utilisation de ces conjugués était déjà envisagée pour des procédés de dosage pouvant être considérés comme se rattachant à ceux décrits dans les demandes de brevets allemands.

Dans le procédé selon l' l'invention, le groupe fixé à l'ATP permet une liaison affine chimique ou immunologique avec une molécule ou un produit lui-même directement révélable ou susceptible d'être révélé à son tour par couplage avec un autre produit révélateur. En outre ce groupe est tel qu'il ne modifie pas la capacité de l'enzyme choisie d'assurer l'incorporation du dérivé modifié d'AMP ou d'ADP portant les mêmes groupes de modification que l'ATP modifié initial, dans ou sur la substance à doser, et ce dans les conditions normales d'incorporation de l'AMP ou de l'ADP non modifié sur la même substance en présence de la même enzyme.

Le procédé selon l'invention de détection ou dosage d'une substance appartenant à la catégorie de substances ci-dessus définie,consiste donc à faire réagir la composition présumée contenir une telle substance avec l'ATP modifié selon l'invention, en présence de l'enzyme spécifique correspondante, dans des conditions permettant l'incorportation d'un AMP ou d'un ADP modifié correspondant, dans ou sur ladite substance et, le cas échéant, après élimination de l'excès d'ATP modifié libre, à placer la composition obtenue dans des conditions permettant l'éventuel couplage des groupes de modification portés par l'AMP modifié ou l'ADP modifié incorporté à ladite substance, avec la molécule ou le produit permettant la reconnaissance du groupe de modification du dérivé initial d'ATP et à détecter ou à doser la susdite

substance éventuellement présente portant lesdits produits de reconnaissance.

Dans le cas d'un aminoglycoside, le dosage mettra par conséquent en oeuvre une réaction qui peut être schématisée par l'équation suivante :

$$\text{R-OH} + \text{ATP-M} \xrightarrow{\text{enzyme spécifique}} \text{R-O-ADP-M+Pi}$$

ou encore pour d'autres substances comme les oses-1-phosphate :

$$\text{R-OH} + \text{ATP-M} \xrightarrow{\text{enzyme spécifique}} \text{R-O-ADP-M+Pi.}$$

Le dosage du composé R-O-AMP-M (ou du composé R-O-ADP-M), après élimination de l'éventuel excès d'ATP-M, est d'une sensibilité considérable. Dans le cas des antibiotiques, cette méthode permet de doser des quantités d'un nanogramme d'antibiotique.

Parmi les substances susceptibles d'être dosées par une telle méthode, on citera, outre les antibiotiques du type aminoglycosides, et à titre d'exemples, les oses-1-phosphate comme le galactose-1-phosphate (étude de la galactosémie) et le glucose 1-phosphate, l'acide N-acétylneuraminique, l'acide 5-phosphoribosyl-1-pyrophosphorique ...

L'enzyme spécifique intervenant dans cette transformation sera en général constituée par une adénylyltransférase, une acylneuraminate citidylyltransférase, une glucose 1-phosphate uridyltransférase, l'ATP phosphoribosyltransférase ....

Le groupe chimique lié de façon covalente à l'ATP peut revêtir les formes les plus diverses, dès lors qu'il possède un groupe permettant son couplage avec des substances affines permettant sa révélation, de préférence sous forme directement visualisable, et qu'il n'empêche pas l'enzyme susdite de catalyser l'incorporation du dérivé d'AMP ou d'ADP correspondant sur la susbtance sus-visée.

Parmi les groupes chimiques préférés susceptibles d'être fixés sur les bases des susdits ribonucléotides, on mentionnera tout groupe susceptible d'être reconnu spécifiquement par une autre molécule ou par un produit, lui-même aisément détectable, de préférence par une méthode de visualisation.

Cette autre molécule ou cet autre produit consiste par exemple en une enzyme, dont la présence peut être révélée par l'action qu'elle est susceptible d'exercer sur un substrat, de préférence un substrat donnant lieu à des réactions de coloration ou de décoloration, ou plus généralement encore de modification de spectres d'absorption respectivement décelables par colorimétrie ou spectrophotométrie. On peut naturellement avoir recours à des molécules ou produits donnant lieu à des réactions de fluorescence, à des modifications de densité optique, etc., par exemple des produits comprenant des groupes dérivés de l'aminofluorène, du chlorure de dansyle, de la rhodamine, etc..

Parmi les groupes de modification appropriés, on mentionnera les groupes chimiques dont est connue l'affinité pour un autre type de molécule chimique. Parmi ces groupes de modification chimique, on peut mentionner la biotine ou l'avidine, dont on connaît les affinités réciproques, les groupes dérivés de l'une de ces molécules pouvant servir à la modification du ribonucléotide choisi et l'autre de molécule couplable avec un réactif marqué par une enzyme ou susceptible d'être fixée à une enzyme, par exemple dans les conditions décrites dans le brevet n° 78 10975 de l'INSTITUT PASTEUR déposé le 13 avril 1978. Ce réactif consiste par exemple en un anticorps spécifique dirigé contre le groupe de modification ou en une molécule ayant une affinité spécifique pour ledit groupe de modification.

Parmi les groupes de modification utiles du ribonucléotide initial, figurent également des antigènes ou haptènes susceptibles d'être reconnus par des anticorps préalablement formés contre ces antigènes ou contre ces haptènes, plus particulièrement lorsque ceux-ci ont été fixés au préalable sur une macromolécule support, telle qu'une sérum-albumine ou un polypeptide, par exemple une polylysine. Parmi ces antigènes ou haptènes, on peut citer la biotine et l'avidine elles-mêmes, des groupes acétylaminofluorène, des peptides, hormones ou prostaglandines, notamment ceux auxquels correspondent des antisérums ou anticorps spécifiques, des lectines, dont est connue la capacité à être couplées à des enzymes permettant leur révélation, notamment des péroxydases, $\beta$-galactosidases, etc... De tels sérums ou anticorps sont disponibles dans le commerce.

Mais la molécule ou le produit présentant des caractéristiques d'affinité vis-à-vis du groupe de modification susdit, sert éventuellement aussi seulement de relais vis-à-vis d'une autre molécule ou d'un autre produit susceptible d'être visualisé à son tour, notamment dans les conditions sus-indiquées ; par

exemple le produit présentant les caractéristiques d'affinité, vis-à-vis du groupe de modification susdit, est constitué par un anticorps lui-même non marqué, mais reconnaissable à son tour par des anticorps contre lui-même, ces derniers anticorps étant eux-mêmes couplés à l'enzyme susceptible d'agir sur un substrat spécifique, dans les conditions classiques en matière de dosage immunoenzymatique.

D'une façon générale, le groupe de modification du ribonucléotide est constitué par toute molécule ou produit chimique fixable sur l'ATP et ensuite détectable dans les conditions qui ont été indiquées, dans la mesure où il répond aux conditions sus-indiquées. L'invention comprend également un test de reconnaissance des groupes compatibles. Ces groupes sont ceux qui n'empêchent pas la fixation de l'AMP modifié correspondant sur des aminoglycosides, notamment la tobramycine en présence d'adénylyl-transférase dans les conditions sus-indiquées, cet AMP modifié devant naturellement être fourni par l'ATP préalablement modifié par le groupe de modification étudié.

Le groupe de modification chimique sus-indiqué est fixé sur la position 6, de préférence 8, du groupe adénine.

Cette fixation intervient avantageusement par l'intermédiaire d'un bras du type -NH-$(CH_2)_x$-X, ou -CO-$(CH_2)_x$-X, dans lequel x varie de 2 à 20, notamment de 6 à 12, et X est un groupe assurant la liaison entre un groupe M, choisi parmi ceux qui sont susceptibles d'une réaction de liaison avec un agent chimique ou immunologique ayant une affinité sélective pour ce groupe. Les groupes $CH_2$ du bras susdit peuvent en partie être remplacés par des groupes CO ou NH, à condition naturellement que ces groupes de remplacement ne soient pas adjacents à des groupes identiques.

Par exemple, si l'on considère le cas de la modification du groupe adénine de l'ATP en sa position 8, le ribonucléotide modifié obtenu peut être représenté par la formule (cas d'un bras du type -NH-$(CH_2)_x$-X-) :

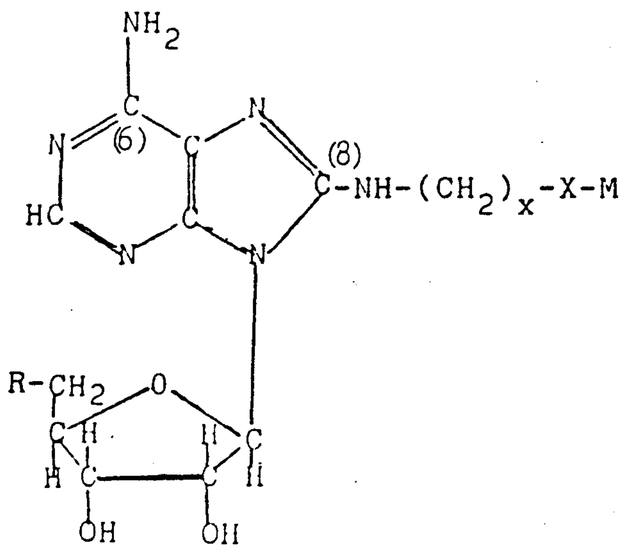

(I)

dans laquelle R est un groupe triphosphate, x, X et M ont les significations sus-indiquées. Avantageusement, le groupe X est constitué par un groupe NH ou CO.

Un procédé pour fabriquer le dérivé de formule I, par exemple à partir de l'ATP préalablement bromé en position 8, consiste à le faire réagir dans les conditions appropriées, avec un composé de formule $H_2N$-$(CH_2)_x$-X-Y, dans lequel Y représente un radical auquel sera ensuite substitué le groupe M susdit, notamment par la mise en oeuvre d'une réaction de condensation avec une molécule MZ, au cours de laquelle est alors formé le produit de condensation de formule I, avec libération d'une molécule Y-Z.

Lorsque le groupe X est NH, Y est avantageusement de l'hydrogène. Lorsque X est CO, Y est avantageusement un hydroxyle. Z peut être constitué par tout groupe susceptible d'être détaché de M dans la susdite réaction de condensation, par exemple le fluor lorsque la molécule chimique donneuse du groupe recherché est le fluoro -1-dinitro-2,4 benzène, ou un hydroxyle ou de l'hydrogène dans le cas d'un peptide. Dans ce dernier cas, la fixation de ce peptide à l'extrémité du bras susdit peut, lorsque le groupe XY est constitué par un groupe $NH_2$ ou COOH, être effectué par la mise en oeuvre de réactions de couplage, traditionnelles dans la chimie des protéines, entre groupes carboxyle et amine, respectivement portés par les deux éléments peptidiques distincts à coupler, par exemple par condensation en présence d'un agent de condensation, tel que le dicyclohexyl-carbodiimide ou après formation préalable d'un ester activé à la fonction carboxylique de celui de ces deux éléments peptidiques qui le portent.

La position 8 sur le cycle adénine de l'ATP ne constitue évidemment pas le seul point sur lequel peut se brancher une chaîne porteuse d'un groupe de modification tel qu'il a été défini plus haut. A titre d'exemple, on peut également substituer l'un des atomes d'hydrogène porté par le carbone en position 6 du cycle adénine par une chaîne porteuse de ce groupe. A titre d'exemple, on mentionnera la possibilité de substitution qui consiste à faire réagir avec l'ATP de l'acide iodoacétique ou un acide organique iodé équivalent, permettant la formation préalable d'un sel quaternaire, faisant intervenir l'azote en position 1, sel qui se transforme ensuite par chauffage en milieu basique à 35 ° C, à pH légèrement basique, notamment à pH 8, pendant le temps suffisant, par exemple 72 heures, en un produit de substitution de l'un des hydrogènes du groupe $NH_2$ fixé sur le carbone en position 6 du groupe adénine (réaction du type connu sous l'expression "réarrangement de Dimroth").

On obtient alors (lorsque l'acide organique iodé est constitué par l'acide iodoacétique) le composé de formule II ci-après :

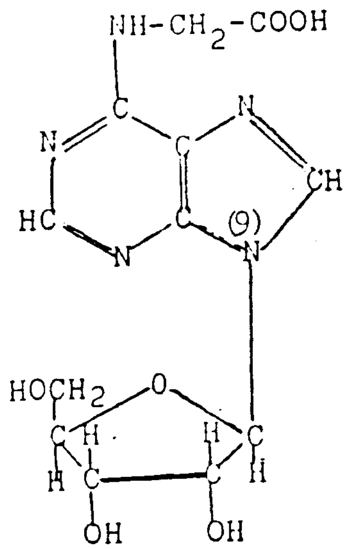

(II)

Ce composé peut ensuite être transformé, par réaction avec le composé de formule $H_2N-(CH_2)_x-X-Y$ déjà défini plus haut, dans des conditions permettant la liaison chimique entre le groupe carbonyle initialement contenu dans le groupe carboxyle dans le composé de formule II et le groupe imino appartenant initialement à la fonction aminée du composé $H_2N-(CH_2)_x-X-Y$, lequel peut alors être couplé à son tour avec un composé de formule MZ, dans les conditions qui ont déjà été définies plus haut.

Il va de soi que tous les éléments qui précèdent ne visent qu'à illustrer des modes de préparation particuliers qui permettent de fixer sur l'ATP un groupe de modification choisi parmi ceux auxquels correspondent des molécules affines, comme cela a été défini plus haut.

D'autres caractéristiques de l'invention apparaîtront encore au cours de la description d'exemples de mises en oeuvre préférées de l'invention.

<u>Préparation du 8-[-N-(dinitro-phényl)-amino-hexyl]-aminoadénosine 5'-triphosphate.</u>

On fait réagit du 8-(aminohexyl)-aminoadénosine 5'-triphosphate avec du fluoro-1-dinitro-2,4-benzène, au sein d'un mélange eau-éthanol 10/1 volumes %, à pH 8,8, à 40 ° C, et en présence d'un sel, notamment chlorure,de magnésium. Le produit de réaction finalement obtenu a pour formule :

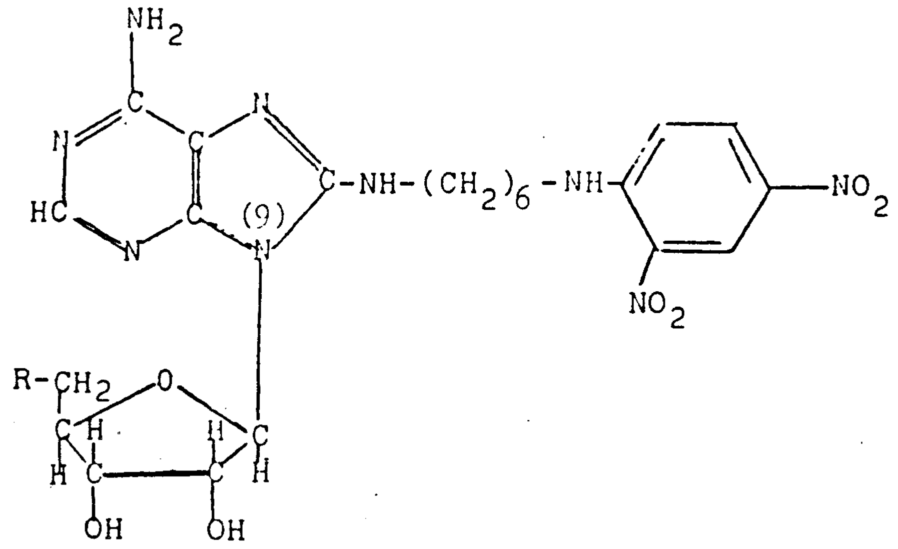

(III)

On récupère le dérivé de formule III, ci-après dénommé ATP-DNP, après une purification comprenant la fixation du ribonucléotide sur DEAE cellulose, élution avec un gradient de LiCl O,2 N, pH 5,5, à LiCl O,5 N, pH 2 et filtration sur tamis moléculaire du type SEPHADEX G50. Le rendement réactionnel est de 52 %. Les fractions recueillies sont analysées par spectrophotométrie d'absorption de rayonnements de longueurs d'onde de 280 et 360 nanomètres respectivement. On recueille celle des fractions dont les densités optiques dans les deux susdits domaines de longueurs d'onde sont dans un rapport ($DO_{280}/DO_{360}$) égal à 4. Le produit contenu dans cette fraction correspond à celui résultant de la fixation de 1 mole de DNP sur 1 mole d'ATP. Il ne donne également qu'une tache dans un système de chromatographie en couche mince. Le produit de cette fraction est lyophilisé.

Ce produit présente la caractéristique d'être reconnu par des anticorps formés au préalable à l'égard de dinitro-2,4 benzène, qui avait préalablement été fixé sur un support macromoléculaire du type de la sérum-albumine. Des anticorps de ce type sont par ailleurs disponibles dans le commerce.

Préparation du 8-(N-biotinyl-aminohexyl)-aminoadénosine 5'-triphosphate.

On condense du 8-(aminohexyl)-amino-adénosine-5'-triphosphate avec le biotinyl-N-hydroxysuccinimideester, dans les conditions décrites par LANCER et al, telles qu'appliquées à la fabrication du biotinyl-UTP à partir de la 5-(3-amino)allyluridine. On obtient alors le composé de formule :

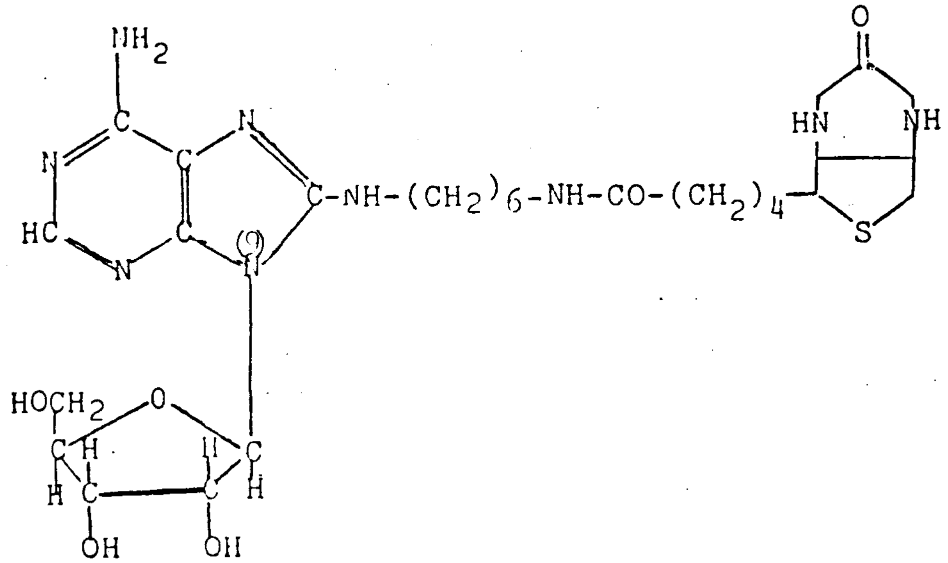

(IV)

Dosage de tobramycine.

L'échantillon présumé contenir le susdit antibiotique est constitué par du sérum provenant d'un patient traité par un aminoglycoside. Ce sérum est préalablement déprotéinisé.

Cet échantillon est mis en solution dans une solution tamponnée dont la composition est la suivante :

| | |
|---|---|
| Tris HCl | 50 mM, pH 7,5 |
| $MgCl_2$ | 10 mM, |
| DTT | 1 mM. |

On ajoute à la solution ainsi formée 10 micromoles d'ATP-DNP et on laisse incuber pendant 1 heure à 37° C avec 10 U d'adénylyltransférase extraite de *Staphilococcus rivet* ou de *Escherichia coli* R55.

Une partie du produit de réaction est alors prélevée, notamment par une pipette d'EPPENDORF (GILSON) comprenant dans sa partie inférieure un échangeur de cations capable de fixer l'antibiotique modifié par l'AMP-DNP, les autres constituants, y compris l'excès d'ATP-DNP et les phosphates organiques formés restant alors dans la solution.

L'échangeur de cations (Amberlite CG 50, 200-400 mcsh, forme $NH_4{+}$) est alors lavé avec la susdite solution tampon. L'antibiotique modifié est ensuite élué à partir de l'échangeur de cations, à l'aide d'une solution d'ammoniaque 1N. Le soluté obtenu est ensuite neutralisé avec HCl 1N.

Le dosage comprend alors finalement l'étape consistant à mettre en contact la susdite solution avec un anticorps anti-DNP couplé à de la péroxydase, à recueillir l'éventuel complexe formé entre, d'une part, l'aminoglycoside dans lequel est incorporé le produit de dégradation de l'ATP-DNP, et, d'autre part, le susdit anticorps et à le mettre en contact avec un substrat colorable de la péroxydase. La présence d'antibiotique est alors manifestée par la coloration du substrat de la péroxydase, coloration qui permet même de fournir des indications quantitatives concernant la quantité d'antibiotique que contenait initialement l'échantillon traité.

Dans l'exemple considéré le substrat est avantageusement constitué par une solution contenant :

- de l'eau oxygénée : 10 $\mu$l d'$H_2O_2$ à 110 volumes,
- de l'acétate de potassium : 9,5 ml O,05M, pH 5,1,
- du 3-amino-9-éthylcarbazole : 2 mg dissous dans O,5 ml de N-N'-diméthylformamide.

On peut par cette méthode doser des quantités de l'ordre du nanogramme par millilitre du susdit aminoglycoside.

L'invention ne se limite évidemment pas aux modes de réalisation décrits ci-dessus à titre d'exemples et l'homme de l'art peut y apporter des modifications sans pour autant sortir du cadre des revendications ci-après.

## Revendications

**1.** Procédé de détection ou de dosage d'une substance appartenant à la catégorie de substances biologiques, telles que certains antibiotiques plus particulièrement ceux de la classe des aminoglycosides, qui sont capables, en présence d'enzymes spécifiques, d'incorporer de l'ADP ou de l'AMP, ces dernières molécules étant susceptibles d'être fournies à ces substances biologiques à partir d'ATP en présence d'une enzyme spécifique appropriée, caractérisé par les étapes qui consistent :

- à mettre la composition présumée contenir la substance recherchée avec un réactif contenant un ATP modifié par une molécule chimique fixée de façon covalente au cycle adénine de cet ATP et portant au moins un groupe non engagé dans la liaison covalente susdite et couplable directement ou indirectement avec une molécule ou un produit ayant une affinité spécifique pour ce groupe et permettant sa reconnaissance, ledit groupe étant en outre tel qu'il n'empêche pas l'ATP modifié qui le porte à fournir l'AMP modifié ou l'ADP modifié correspondant dans une forme incorporable dans ladite substance recherchée.
- à placer, le cas échéant, après élimination des ATP modifiés libres, la composition obtenue dans des conditions permettant l'éventuel couplage des groupes de modification portés par l'AMP modifié ou l'ADP modifié incorporé à ladite substance, avec la molécule ou le produit permettant la reconnaissance du groupe de modification du dérivé initial d'ATP, et
- à détecter ou à doser la susdite substance éventuellement présente portant lesdits produits de reconnaissance.

**2.** Procédé selon la revendication 1, caractérisé en ce que le réactif utilisé contient un ATP modifié résultant de la fixation covalente sur la position 6, ou de préférence 8, du groupe adénine de l'ATP d'un groupe M, choisi parmi ceux qui sont susceptibles d'une réaction de liaison avec un agent chimique ou

immunologique ayant une affinité sélective pour ce groupe, par l'intermédiaire d'un bras du type -NH-$(CH_2)_x$-X, ou -CO-$(CH_2)_x$-X, dans lequel X est un groupe assurant la liaison avec le susdit groupe M et x varie de 2 à 20, notamment de 6 à 12.

**3.** Procédé selon la revendication 1, caractérisé en ce que le susdit groupe M est susceptible d'être reconnu spécifiquement et directement par une autre molécule ou par un produit, lui-même aisément détectable, par une méthode de visualisation.

**4.** Procédé selon la revendication 3, caractérisé en ce que le susdit groupe M est constitué par un antigène ou un haptène susceptible d'être reconnu par un anticorps préalablement formé contre cet antigène ou contre cet haptène.

**5.** procédé selon la revendication 2, caractérisé en ce que le susdit groupe M sert de relais vis-à-vis d'une autre molécule ou d'un autre produit susceptible d'être visualisé à son tour.

**6.** Procédé selon l'une quelconque des revendications 2 à 4, caractérisé en ce que le susdit groupe M est couplable chimiquement avec une molécule affine marquée par une enzyme ou immunologiquement avec un anticorps marqué par une enzyme et ayant une affinité sélective pour ledit groupe.

**7.** Procédé selon la revendication 2 considérée isolément ou en combinaison avec l'une quelconque des revendications 3 à 6, caractérisé en ce que les groupes $CH_2$ du bras susdit peuvent en partie être remplacés par des groupes CO ou NH, à condition que ces groupes de remplacement ne soient pas adjacents à des groupes identiques.

**8.** Procédé selon l'une quelconque des revendications 2 à 7, caractérisé en ce que le susdit groupe M est dérivé de l'avidine ou un groupe dinitro-2,4-phényle.

**9.** Procédé selon l'une quelconque des revendications 2 à 7, caractérisé en ce que le susdit groupe M est dérivé de l'avidine.

**10.** Procédé selon l'une quelconque des revendications 2 à 7, caractérisé en ce que le susdit groupe M est un groupe dinitro-2,4-phényle.

## Claims

**1.** Procedure for the detection or assay of a substance belonging to the set of biological substances, such as certain antibiotics and more particularly those of the aminoglycoside class, which are capable, in the presence of specific enzymes, of incorporating ADP or AMP, these latter molecules being capable of being supplied to these biological substances from ATP in the presence of a suitable specific enzyme, characterized by the steps which consist of:

- placing the composition presumed to contain the desired substance in contact with a reagent containing an ATP modified by a chemical molecule covalently attached to the adenine ring of this ATP and bearing at least one group not involved in the above-mentioned covalent linkage and which can be coupled directly or indirectly with a molecule or a product which has a specific affinity for this group and which makes possible its recognition, the said group being, in addition, such that it does not prevent the modified ATP which bears it from supplying the corresponding modified AMP or modified ADP in a form in which it can be incorporated into the desired said substance.
- placing, if necessary after removal of the free modified ATPs, the composition obtained under conditions which may lead to the possible coupling of the modifying groups borne by the modified AMP or modified ADP incorporated into the said substance with the molecule or product which makes possible the recognition of the modifying group of the initial ATP derivative, and
- detecting or assaying the above-mentioned substance possibly present bearing the said recognition products.

**2.** Procedure according to Claim 1, characterized in that the reagent used contains a modified ATP resulting from the covalent linking at position 6, or preferably position 8,of the adenine group of the ATP of a group M, selected from those which are capable of a binding reaction with a chemical or

immunological agent which has a selective affinity for this group through the intermediary of an arm of the type -NH-$(CH_2)_x$-X or -CO-$(CH_2)_x$-X, in which X is a group which forms the linkage to the above-mentioned M group and x varies from 2 to 20, and in particular from 6 to 12.

**3.** Procedure according to Claim 1, characterized in that the above-mentioned group M is capable of being recognized specifically and directly by another molecule or by a product, itself readily detectable by a method of visualization.

**4.** Procedure according to Claim 3, characterized in that the above-mentioned group M is constituted by an antigen or a hapten capable of being recognized by an antibody formed beforehand against this antigen or against this hapten.

**5.** Procedure according to Claim 2, characterized in that the above-mentioned group M serves as relay for another molecule or another product capable of being visualized in its turn.

**6.** Procedure according to any one of the Claims 2 to 4, characterized in that the above-mentioned group M can be coupled chemically with an affinity molecule labelled by an enzyme or immunologically with an antibody labelled with an enzyme and having a selective affinity for the said group.

**7.** Procedure according to Claim 2 considered in isolation or in combination with any one of the Claims 3 to 6, characterized in that the $CH_2$ groups of the above-mentioned arm can be replaced, in part, by CO or NH groups, provided that these substituent groups are not adjacent to groups of the same type.

**8.** Procedure according to any one of the Claims 2 to 7, characterized in that the above-mentioned group M is derived from avidin or a 2,4-dinitrophenyl group.

**9.** Procedure according to any one of the Claims 2 to 7, characterized in that the above-mentioned group M is derived from avidin.

**10.** Procedure according to any one of the Claims 2 to 7, characterized in that the above-mentioned group M is a 2,4-dinitrophenyl group.

**Patentansprüche**

**1.** Verfahren zum Nachweis oder zur Bestimmung einer Substanz aus einer Gruppe biologischer Substanzen, wie bestimmte Antibiotika, insbesondere solche aus der Klasse der Aminoglykoside, die in Gegenwart spezifischer Enzyme ADP oder AMP einbauen können, wobei diese Moleküle den biologischen Substanzen aus ATP in Gegenwart eines geeigneten spezifischen Enzyms bereitgestellt werden können, dadurch **gekennzeichnet**, daß man
- die Zusammensetzung, die vermutlich die gesuchte Substanz enthält, mit einer reaktiven Verbindung umsetzt, welche ein ATP enthält, das durch ein kovalent am Adeninring dieses ATPs gebundenes chemisches Molekül modifiziert ist, und mindestens eine nicht an der kovalenten Bindung beteiligte Gruppe enthält und direkt oder indirekt mit einem Molekül oder einem Produkt koppelbar ist, das eine spezifische Affinität für diese Gruppe besitzt und ihre Erkennung gestattet, wobei diese Gruppe überdies derart beschaffen ist, daß sie nicht verhindert, daß das modifizierte ATP, das sie trägt, modifiziertes AMP oder modifiziertes ADP entsprechend einer in die gesuchte Substanz einbaubaren Form bereitstellt,
- gegebenenfalls nach Entfernung der freien modifizierten ATPs die Zusammensetzung, die unter Bedingungen erhalten worden ist, die die fakultative Kopplung von Modifikationsgruppen am modifizierten AMP oder modifizierten ADP, die in diese Substanz eingebaut sind, gestatten, zu dem Molekül oder dem Produkt gibt, das die Erkennung einer Modifikationsgruppe des anfänglichen ATP-Derivats gestattet, und
- die gegebenenfalls vorhandene Substanz, die die Erkennungsprodukte trägt, nachweist oder bestimmt.

**2.** Verfahren nach Anspruch 1, dadurch gekennzeichnet, daß die verwendete reaktive Verbindung ein modifiziertes ATP enthält, das aus der kovalenten Bindung eines Restes M, ausgewählt aus solchen, die eine Bindungsreaktion mit einer chemischen oder immunologischen Verbindung mit einer selektiven

Affinität für diesen Rest eingehen können, über einen Spacer vom Typ -NH-$(CH_2)_x$-X oder -CO-$(CH_2)_x$-X, worin X ein Rest ist, der die Bindung mit dem Rest M gewährleistet und x den Wert 2 bis 20, insbesondere 6 bis 12 hat, an Position 6, oder bevorzugt 8, der Adeningruppe von ATP stammt.

**3.** Verfahren nach Anspruch 1, dadurch gekennzeichnet, daß der Rest M spezifisch und direkt von einem anderen Molekül oder einem Produkt, das selbst leicht nachweisbar ist, nach einem Verfahren zur Sichtbarmachung nachgewiesen werden kann.

**4.** Verfahren nach Anspruch 3, dadurch gekennzeichnet, daß der Rest M aus einem Antigen oder Hapten besteht, das durch einen Antikörper, der zuvor gegen dieses Antigen oder gegen diese Hapten erzeugt worden ist, erkannt werden kann.

**5.** Verfahren nach Anspruch 2, dadurch gekennzeichnet, daß der Rest M dem Austausch gegen ein anderes Molekül oder ein anderes Produkt, das seinerseits sichtbar gemacht werden kann, dient.

**6.** Verfahren nach einem der Ansprüche 2 bis 4, dadurch gekennzeichnet, daß der Rest M chemisch an ein verwandtes Molekül, das durch ein Enzym markiert ist, oder immunologisch an einen Antikörper, der durch ein Enzym markiert ist und eine selektive Affinität für diesen Rest besitzt, koppelbar ist.

**7.** Verfahren nach Anspruch 2 allein betrachtet oder zusammen mit einem der Ansprüche 3 bis 6, dadurch gekennzeichnet, daß die Gruppen $CH_2$ des Spacers teilweise durch die Gruppen CO oder NH ersetzt sein können, mit der Maßgabe, daß die Ersatzgruppen nicht neben identischen Gruppen stehen.

**8.** Verfahren nach einem der Ansprüche 2 bis 7, dadurch gekennzeichnet, daß der Rest M sich von Avidin oder einer 2,4-Dinitrophenylgruppe ableitet.

**9.** Verfahren nach einem der Ansprüche 2 bis 7, dadurch gekennzeichnet, daß der Rest M ein Avidinderivat ist.

**10.** Verfahren nach einem der Ansprüche 2 bis 7, dadurch gekennzeichnet, daß der Rest M eine 2,4-Dinitrophenylgruppe ist.